# EUROPEAN PATENT APPLICATION

(11) **EP 1 013 756 A1**
(43) Date of publication of application: **28.06.2000**
(21) Application number: 98403235.9
(22) Date of filing: 21.12.1998
(51) Int. Cl.: C12M 3/04

(54) **An apparatus used to hold and grow cells and allow their processes to grow and interconnect the individual cells and a method for making the apparatus**

(71) Applicant: CORNING INCORPORATED, Corning, N.Y. 14831 (US)
(72) Inventor: Dannoux, Thierry Luc Alain, Corning Incorporated, Corning, NY 14831 (US); Root, David, Corning Incorporated, Corning, NY 14831 (US); Geahel, Isabelle Jeannette M., Corning Inc., Corning, NY 14831 (US)
(74) Representative: Ertl, Nicholas Justin

(57) **Abstract**

An apparatus or device that can hold and grow individual cells and allow their processes to grow connect the cells in accordance with the present invention includes a plate, a plurality of valleys in one surface of the plate, and a plurality of intersecting regions at each location where one of the valleys crosses another one of the valleys. The plate is made of a thermoformable material. Each of the valleys provides sufficient room for the growth of processes from one or more of the cells, but not enough room for one of the cells to fit in. Each of the intersecting regions provides sufficient room for one of the cells to fit in.

## Description

### FIELD OF THE INVENTION

The present invention relates to an apparatus that can hold and grow cells and allow their processes to grow and interconnect the cells, such as nerve cells, to form a neural network. The invention also relates to a method for making this apparatus.

### BACKGROUND OF THE INVENTION

Biological research labs are doing a variety of different work related to nerve cells, including the understanding of communication between nerve cells and attempts to find methods and/or drugs which will allow nerve cells to regenerate after damage by disease or trauma. To perform research work related to the understanding of how nerve cells communicate and how nerve cells can be regenerated requires in vitro methods and devices. In particular, a device is needed which can hold and grow individual nerve cells and allow their processes to grow and interconnect the cells into a neural network. To date, no such device exists.

One existing device used in biological testing is a tray which simply provides a substantially flat surface or plane. One example of such a device is disclosed in U.S. Patent No. 5,240,854 to Berry et al. which is herein incorporated by reference. Although these trays are useful for some tests, these trays will not provide the proper separation for the processes of the nerve cells to interconnect.

Another existing device used in biological testing is a tray with a plurality of isolated wells on one surface of the tray. Some examples of these types of trays are disclosed in U.S. Patent No. 4,599,315 to Terasaki et al., U.S. Patent No. 5,210,021 to Goodwin, Jr., U.S. Patent No. 5,252,294 to Kroy et al.,U.S. Patent No. 5,534,227 to Lahm et al., and U.S. Patent No. 5,540,891 to Portman et al. which are all herein incorporated by reference. Although these trays can be effectively used to grow and tests cells in a nutritive medium in each isolated well, they can not be used to foster growth of processes between wells to generate interconnections to form a neural network.

Another existing device is a microculture tray consisting of a plurality of pairs of wells with each pair of wells connected by a trough which extends downwardly about two-thirds of the depth of the well so that the wells in the pair are connected to allow free passage of supernatant, but not of the cells. One example of such a tray is disclosed in U.S. Patent No. 5,422,270 to Caspi. Again like the previously describe trays, this tray does not permit growth of processes between individual cells. Additionally, only sets of two wells are even partially connected together.

### SUMMARY OF THE INVENTION

An apparatus or device that can hold and grow individual cells and allow their processes to grow connect the cells in accordance with the present invention includes a plate, a plurality of valleys in one surface of the plate, and a plurality of intersecting regions at each location where one of the valleys crosses another one of the valleys. The plate is made of a thermoformable material. Each of the valleys provides sufficient room for the growth of processes from one or more of the cells, but not enough room for one of the cells to fit in. Each of the intersecting regions provides sufficient room for one of the cells to fit in.

A method of manufacturing the apparatus or device for holding and growing cells and promoting lateral growth of processes between the cells includes several steps. First, a plate made of a thermoformable material and a mold are heated to a first temperature. The mold has a plurality of overlapping, wall-shaped protrusions extending up from a base. Next, one surface of the plate and the plurality of overlapping, wall-shaped protrusions extending up from the base of the mold are pressed together. The plurality of overlapping, wall-shaped protrusions form valleys and intersecting regions in the one surface. The valleys provide sufficient room for the growth of processes from one or more of the cells, but not enough room for one of the cells to fit in and the intersecting regions each provide sufficient room for one of the cells.

The apparatus provides a number of advantages including providing a biological test apparatus which can hold and grow individual cells and allow their processes to grow and connect the individual cells into a neural network. The apparatus is designed so that a single cell fits in each intersecting region, but only the processes from these cells fit in the valleys between the intersecting regions. The apparatus is designed to promote lateral growth and provides proper separation for the formation of a neural network. The resulting neural networks can be studied and can be used to repair nerve cells damaged by disease or trauma.

Another advantage is that the apparatus is relatively easy and inexpensive to manufacture. The manufacturing process uses well known and low cost photolithography and chemical etching processes. Once the appropriate mold has been etched, the mold simply needs to be heated with a plate to a softening temperature for the plate and then the plate and mold are pressed together. The depth at which the mold is pressed into the glass is controlled so that the mold does not contact the regions between the valleys being formed to preserve the initial glass quality of the device in those regions. As a result, the cells and processes can not attach to these regions between the valleys.

### BRIEF DESCRIPTION OF THE DRAWINGS

FIG. 1 is a top view of an apparatus that can hold, grow, and interconnect cells in accordance with one embodiment of the present invention;
FIG. 2 is a cross-sectional view of the apparatus taken along line 2-2 in FIG. 1;
FIG. 3 is a top view of a mold used to form the device;
FIG. 4 is a cross-sectional view of the mold taken along line 4-4 in FIG. 3;
FIG. 5 is a cross-sectional view of the mold in contact with the apparatus;
FIG. 6 is a cross-sectional view of several molds in contact with several plates;
FIG. 7 is a picture of a top view of the mold; and
FIG. 8 is a picture of a top view picture of the apparatus

### DETAILED DESCRIPTION

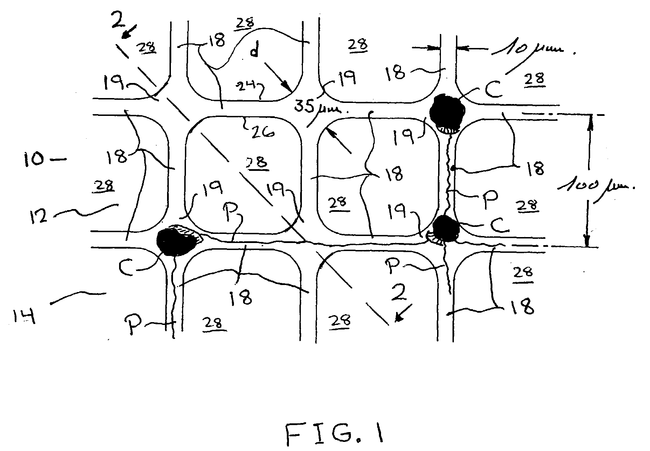

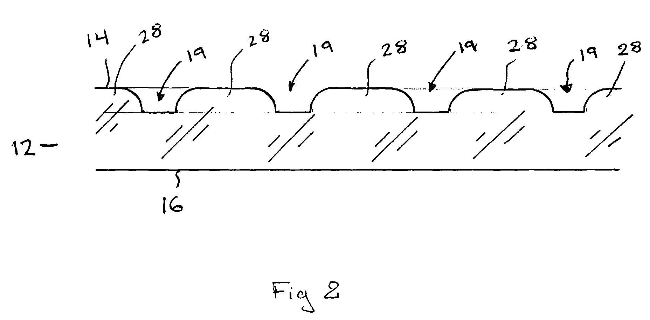

An apparatus or device 10 that can hold, grow, and interconnect cells in accordance with one embodiment of the present invention is illustrated in FIGS. 1, 2, and 8. The apparatus includes a plate, a plurality of valleys on one surface of the plate, and a plurality of intersecting regions at each location where one of the valleys crosses another one of the valleys. The plate provides a number of advantages including providing a biological test apparatus which can hold and grow individual cells and allow the processes to grow and connect the individual cells into a neural network. Another advantage is that the apparatus is relatively easy and inexpensive to manufacture.

Referring more specifically to FIGS. 1, 2, and 8, the plate 12 has a pair of opposing surfaces 14 and 16. The plate 12 is made of a thermoformable material, such as glass. By way of example, in this particular embodiment the plate 12 is made of Coming's Coming Code 0211.

A plurality of valleys 18 are formed in one of the surfaces 14 of the plate 12. Each valley 18 has a bottom region 20 and a top region 22 which is the region between the opposing sides of each valley 18. The valleys 18 each have a width and depth which is sufficiently large to permit processes P from individual cells C to grow and interconnect with other cells C, but which is not large enough to permit a cell C to fit in one of the valleys 18. By way of example, in this particular embodiment the width of the bottom region 20 ranges between about five to ten micrometers, the width of the top region 22 ranges between about ten to twenty micrometers, and the depth of the valley from the top region to the bottom region 20 are substantially uniform throughout, ranging between about 10 and 15 micrometers, although these dimensions can vary as needed or desired. The regions 28 of the plate 12 between the valleys 18 are surface modified to prevent attachment of cells C or processes P while the valleys 18 are designed to foster lateral growth of processes P and the intersecting regions 19 are designed to foster growth of individual cells C. Although the valleys 18 are shown on one surface 14 of the plate 12, valleys 18 can be formed on both of the opposing surfaces 14 and 16.

The intersecting region 19 is formed at each location where the valleys 18 overlap. In this particular embodiment, the valleys 18 overlap in a square-shaped pattern, although the pattern of how the valleys 18 overlap can vary as needed or desired. The intersecting regions 19 connected by the valleys 18 are spaced at distances appropriate to form a group of interconnected cells C, such as a neural network. The intersecting regions 19 each have a width and depth which is sufficiently large to receive a cell C, as well as any other necessary nutritive materials. By way of example, in this particular embodiment each of the intersecting regions 19 have a width of about 30 to 40 micrometers and a depth ranging between about 10 to 15, although these dimensions can vary as needed or desired.

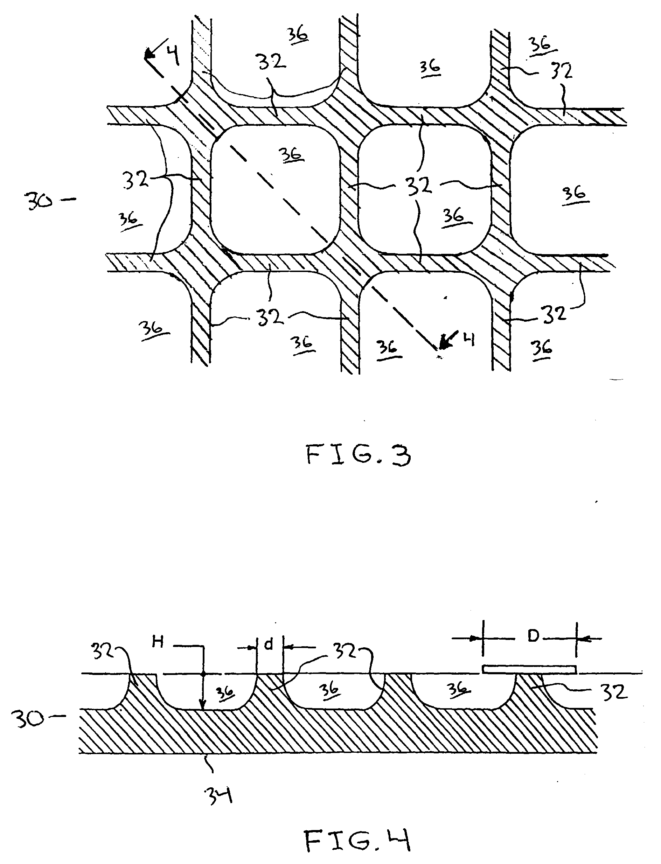

Referring to FIGS. 3, 4, and 7, a mold 30 used to manufacture the apparatus 10 is illustrated. The mold 30 has a plurality of overlapping, wall-shaped protrusions 32 extending up from a base 34 which are arranged in the pattern in which the valleys and intersecting regions are to be formed in the one surface 14 of the plate 12. The mold 30 has a plurality of wells 36 between the wall-shaped protrusion. The mold also has a width of d for each protrusion 32 at the upper surface of the mold 30, a height of H for each protrusion 32, and a width D which is the diameter of the masked surface of the mold pzioz to chemical etching. By way of example, in this particular example the protrusions 32 on the mold 30 have a height H ranging between about 25 and 35 micrometers, a width d for the top of the protrusions 32 ranging between about 60 and 80 micrometers, and a length between points where the protrusions 32 intersect ranging between about 150 and 300 micometers, although the dimensions can vary as needed or desired. The protrusions 32 have a depth or height which is greater than the depth of the valleys 18 so that when the mold 30 and plate 12 are pressed together, the mold 30 stops before it contacts the regions 28 of the plate between the valleys 18. This helps to preserve the initial quality of the plate 12 in these regions 28 between the valleys 18 so that cells C and processes P do not attach to these regions 28 and remain in the valleys 18 and intersecting regions 19.

In order to produce the device for holding, growing, and interconnecting cells according to the present invention, the mold 30 must first be generated. The mold 30 is made by first selecting a sheet of material of some suitable stable superalloy, such as Inconel 600 from Hamilton Precision Metaling. Stable means that the dimensions of the mold 30 do not change when cycled through a temperature change of for example 20°C to 720°C to 20°C. Next, one of the surfaces of the material is covered with a photosensitive resin. A mask which represent the desired pattern for the surface of the mold 30 is placed over the resin and then the surface of the material covered with resin is exposed to light. The exposed areas of resin are then stripped away, leaving a pattern of resin on the surface in the desired pattern for the mold 30.

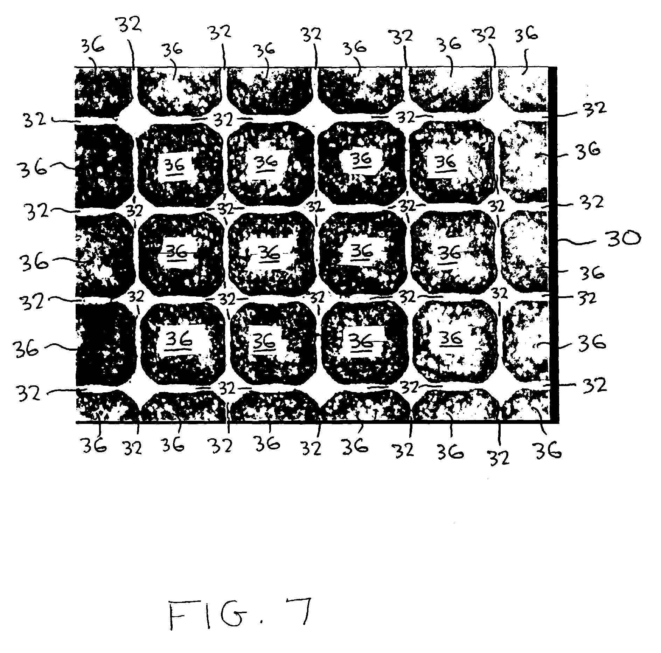

Next, the mold 30 is chemically etched through this resin mask using an etching solution which does not attack the alloy under the remaining areas of resin. In this particular embodiment, the chemical etching is conducted in a FeCl³ etching bath with 20-30 percent HNO₃ acid. The bath attacks and dissolves the metal which is not covered by the resin. The mold 30 is removed from the etching bath and the remaining resin is removed with a suitable solvent. One example of the resulting mold 30 made using this process is illustrated in FIG. 7.

Once the mold 30 is completed, a plate 12 made of thermoformable material, such as Corning's Corning Code 0211, is selected to be used to form the apparatus 10 for holding, growing, and interconnecting cells according to the present invention. The mold 30 and the plate 12 are heated to a softening temperature for the plate 12 and are kept there until the temperature stabilization is obtained. In this particular embodiment, the softening temperature for the plate 12 is 645°C and the glass viscosity is then 10^{g,8} poises, although this temperature and viscosity can vary depending upon the material selected for the plate 12.

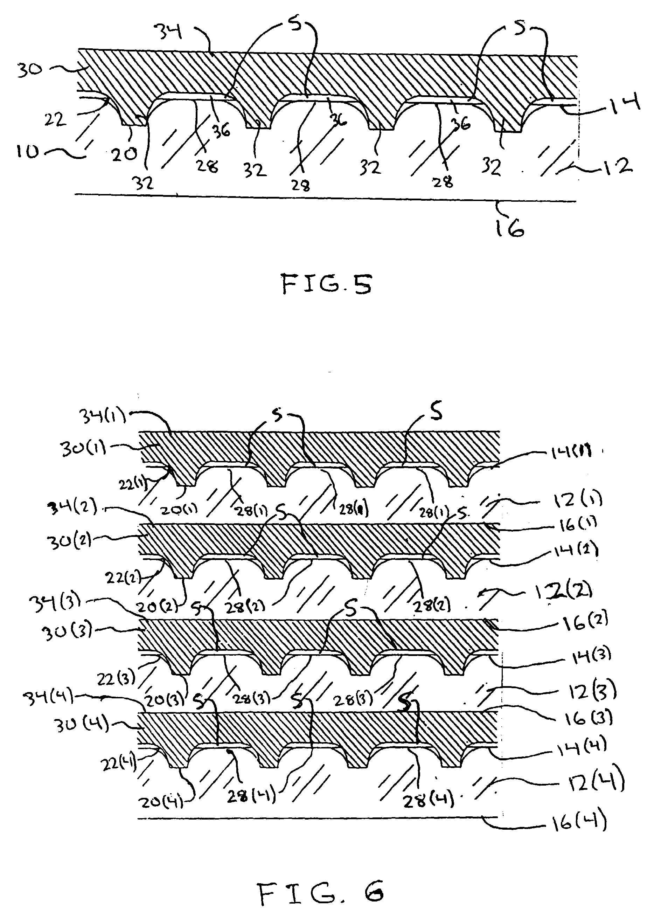

Next as shown in FIG. 5, the plate 12 and mold 30 are pressed together. Either the mold 30 can be pressed into one surface 14 of the plate 12 or the plate 12 can be pressed into one surface of the mold 30 with the protrusions 32. The protrusions 32 extend into one surface of the plate 12 to form the valleys 18 and intersecting regions 19. The pressing procedure is controlled so that the base of the wells 36 of the mold 30 between the protrusions 32 do not come into contact with the regions 28 between the valleys 18 being formed on the plate 12. A space S is left between the base of the wells 36 and the regions 28 between the valleys 18. As shown in FIG. 6, a plurality of molds 30(1)-30(4) and plates 12(1)-12(4) can be heated and then pressed together at the same time to increase the volume and to farther reduce the cost at which these devices can be manufactured.

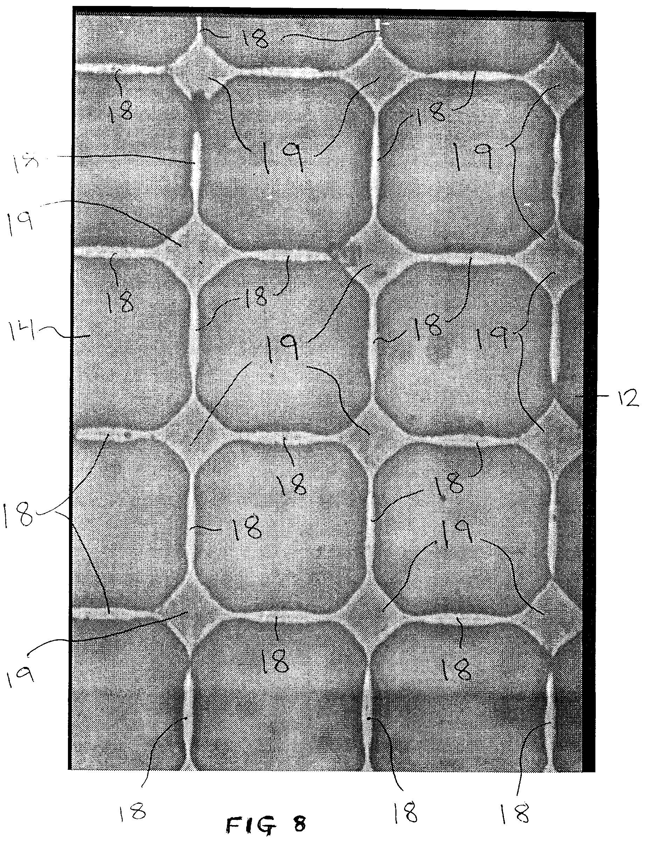

Once the desired depth for the protrusions 32 into one surface 14 of the plate 12 has been reached, the mold 30 and plate 12 are held together until the plate 12 solidifies. In this particular embodiment, the mold 30 and plate 12 are held together until the mold 30 and plate 12 reach room temperature. At this point, the mold 30 is removed from the plate 12 and the apparatus 10 is completed. As illustrated and discussed above, the manufacturing process is easy and low cost, utilizing well know photolithography and chemical etching process to make the mold 30 and easy steps to make the apparatus 10. One example of the apparatus 10 made using this process is illustrated in FIG. 8. Although one method for manufacturing the apparatus 10 to hold, grow, and interconnect cells C is illustrated and discussed, other methods to manufacture, such as rolling, blowing, or suction of the material against mold 30 could be used to make the apparatus 10.

Having thus described the basic concept of the invention, it will be rather apparent to those skilled in the art that the foregoing detailed disclosure is intended to be presented by way of example only, and is not limiting. Various alterations, improvements, and modifications will occur and are intended to those skilled in the art, though not expressly stated herein. These alterations, improvements, and modifications are intended to be suggested hereby, and are within the spirit and scope of the invention. Accordingly, the invention is limited only by the following claims and equivalents thereto.

## Claims

1. An apparatus which holds and grows cells and promotes lateral growth of processes between the cells, the apparatus comprising:
a plate having a first surface;
a plurality of valleys formed in the first surface, wherein each of the valleys provides sufficient room for the growth of processes from one or more of the cells, but not enough room for one of the cells to fit in; and
an intersecting region at each location where one of the valleys crosses another one of the valleys, wherein each of the intersecting regions provides sufficient room for one of the cells to fit in.

2. The apparatus as set forth in claim 1 wherein each of the valleys has a bottom region with a width ranging between about five to ten micrometers and a top region defined between opposing sides of the first surface having a width ranging between about ten to twenty micrometers.

3. The apparatus as set forth in claim 1 wherein each of the intersecting regions has a width ranging between about sixty to eighty micrometers.

4. The apparatus as set forth in claim 1 wherein the plate is made from glass.

5. The apparatus as set forth in claim 1 wherein the valleys are arranged in a square-shaped crossing pattern.

6. A method of manufacturing a device which holds and grows cells and promotes lateral growth of processes between the cells, the method comprising the steps of:
heating a plate made of a thermoformable material to a first temperature;
heating a mold to the first temperature, the mold having a plurality of overlapping, wall-shaped protrusions extending up from a base; and
pressing one surface of the plate and the plurality of overlapping, wall-shaped protrusions extending up from the base of the mold together.

7. The method according to claim 6 wherein the plurality of overlapping, wall-shaped protrusions extending up from the form valleys and intersecting regions in the one surface of the plate, the valleys providing sufficient room for the growth of processes from one or more of the cells, but not enough room for one of the cells to fit in and the intersecting regions provides sufficient room for one of the cells

8. The method according to claim 7 wherein each of the valleys formed in the one surface of the plate have a bottom region with a width ranging between about five to ten micrometers and a top region defined between opposing sides of the one surface having a width ranging between about ten to twenty micrometers.

9. The method according to claim 7 wherein each of the intersecting regions has a width ranging between about thirty to forty micrometers.

10. The method according to claim 6 wherein the wall-shaped protrusions are pressed into the one surface of the plate to a depth which is a less than the height of the protrusions.

11. The method according to claim 6 wherein the first temperature is the softening temperature for the thermoformable material.

12. The method according to claim 11 wherein the thermoformable material is glass.

13. The method according to claim 6 further comprising the step of etching a first material to form the mold having the plurality of overlapping, wall-shaped protrusions extending up from the base.

14. The method according to claim 13 wherein the first material is a metal.
